# EUROPEAN PATENT APPLICATION

(11) **EP 3 599 031 A1**
(43) Date of publication of application: **29.01.2020**
(21) Application number: 18185741.8
(22) Date of filing: 26.07.2018
(51) Int. Cl.: B05C 17/005

(54) **DISCHARGER, CONTAINER, DISPENSING PART, ACTIVATION PART AND METHOD OF ASSEMBLING AND USING A DISCHARGER**

(71) Applicant: Sulzer Mixpac AG, 9469 Haag (Rheintal) (CH)
(72) Inventor: Noack, Michaela, 6890 Lustenau (AT); Eggmann, Marc, 8865 Bilten (CH)
(74) Representative: Manitz Finsterwald Patent- und Rechtsanwaltspartnerschaft mbB

(57) **Abstract**

The present invention relates to a discharger (10), the discharger comprising a two-part housing (12) formed of a dispensing part (14) and an activation part (16) and further comprising a container (18) configured to store a fluid in a storage state of the discharger. The invention further relates to a container, to a dispensing part, to an activation part as well as to a method of assembling and using a discharger.

## Description

The present invention relates to a discharger, the discharger comprising a two-part housing formed of a dispensing part and an activation part and further comprising a container configured to store a fluid in a storage state of the discharger. The invention further relates to a container, to a dispensing part, to an activation part as well as to a method of assembling and using a discharger.

Dischargers are used to dispense pre-defined amounts of fluids from a container associated with the discharger. The pre-defined amounts are typically composed of single doses of e.g. a medication. On use of the discharger the container is moved towards a static piston and the seal of the container is pierced in order to permit the fluid stored in the container to be discharged out of the container and from the discharger via the static piston and a dispensing element.

During the piercing action, the static piston arranged within a housing of the discharger initially engages the seal of the container resulting in a pressure on the seal of the container leading to an increase in pressure of the fluid stored therein, prior to the actual piercing of the seal. Once pierced, this then pressurized fluid, particularly if it is a low viscous fluid, such as a tick or flea drug for animals, can be discharged from the container via the static piston. Particularly on use of low viscous fluids particular attention has to be paid on the seals present within the discharger to avoid a person handling the discharger from coming into non-desired contact with the fluid stored in the container due to leaks present in the discharger. For this reason prior art dischargers are typically designed for single-dose applications and hence for one time use. This leads to considerable waste, since the complete discharger is then disposed of after use.

In view of this background it is an object of the invention to make available a discharger in which the material waste is reduced after use of the discharger. It is a further object of the present invention to ensure a correct installation of the container at the discharger to minimize the risk of leaks due to a faulty alignment of the components of the discharger. It is yet a further object of the invention to minimize a leakage of the fluid contained in the container from the discharger while the discharging takes place.

This object is satisfied by a discharger in accordance with claim 1.

Such a discharger, preferably a manually operated discharger for single dose applications, comprises a two-part housing formed of a dispensing part and an activation part and further comprises a container configured to store a fluid in a storage state of the discharger, the container being stored within a compartment formed between the dispensing and activation parts, wherein the dispensing part and the activation part are configured to be releasably coupled to one another by means of a releasable connection, with the dispensing part comprising a static piston and the activation part comprising a reception space forming at least part of the compartment configured to removably store the container in the storage state of the discharger, the discharger further comprising an actuation device that cooperates with the container and that is configured to move the container from the storage state into a discharge state in which the fluid stored in the container can be discharged via the static piston.

Forming a discharger such that it has a releasable connection between the dispensing part and the activation part and that has a compartment present between the activation part and the dispensing part that is configured to removably store a container between the dispensing and activation parts makes available a discharger of which at least some parts can be re-used.

By way of example, those parts of the discharger that come into contact with the fluid stored in the container, such as the dispensing part and/or the container may be formed as disposable parts that are respectively configured for a one time use. Moreover, other parts of the discharger such as the activation part - that should not come into contact with the fluid stored in the container - may be a reusable part configured for multiple uses.

A releasable connection is a connection that can be opened and closed without tools, i.e. by hand.

Moreover, a releasable connection is a connection that can be opened in a nondestructive manner, i.e. on releasing the connection between the dispensing and activation parts, neither one of the dispensing and activation parts nor the container are destroyed.

Further, providing an actuation device that is capable of moving the container stored in the housing of the discharger from a storage state into a discharge state makes available a means for activating the discharger.

In this connection it should be noted that the storage state is a state in which the container is configured to store a fluid, without the fluid being able to leave the container. For this purpose the container comprises a seal that is closed in the storage state and that can be opened on activation of the discharger by means of the static piston arranged in the dispensing part on moving the container from the storage state into the discharge state.

A prior art single dose nasal dispenser is e.g. known from US 5,431,155, this does not comprise a releasable connection and this does not contain a separate container so that this device is not reusable and no waste can be saved on using this prior art dispenser in comparison to the discharger of the present invention.

The actuation device and/or the activation part may comprise alignment means that are configured to permit the correct alignment of the container in the discharger on installation of the container in the discharger. Thereby a misalignment of the container in the discharger can be avoided as can a source of leaks.

Exemplary types of the releasable connection are formed by at least one of a bayonet type of connection, a plug and rotate type of connection, a Luer lock type of connection, and a threaded connection. In this way a comparatively quick connection and release of the dispensing part and the activation part is made available.

It is preferred if a latched connection is present between the dispensing and activation parts to fix the dispensing and activation parts one to another on establishing the connection between the two parts, wherein the latched connection is in particular formed at the releasable connection and can be released on opening the releasable connection.

Advantageously the dispensing part and the activation part each comprise two slots, with respective parts of the slots of the dispensing and activation parts cooperating with one another and forming the releasable connection between the dispensing and activation parts. Thereby a plug and rotate connection is made available that enables a secure and quick release and/or closing of the releasable connection on assembling and/or disassembling the discharger.

In this connection it should be noted that a plug and rotate type of connection is a connection between two parts that are connected one to another by means of a connection method that comprises an axial and a rotational movement of the two parts relative to one another.

Optionally the slots may be inclined with respect to a longitudinal axis of the discharger that is aligned with the static piston. This further ensures a correct installation of the container at the discharger.

Preferably at least one of the slots of the dispensing and activation parts comprises a recess in one of its walls cooperating with a projection formed at a wall of the respective other one of the dispensing and activation parts to form the latched connection.

It is preferred if the actuation device comprises at least one button or lever that is configured to be pressed to bring about a movement of the container from the storage state into the discharge state, preferably wherein the container remains in the discharge state after discharging the fluid previously stored in the container. In this way one can ensure that on opening the releasable connection between the activation part and the dispensing part that the container remains at a dispensing end of the activation part to ensure that the activation part does not come into contact within any fluid remaining in the container.

At least a part of the actuation device may be configured to be moved relative to the activation part, i.e. at least some of the second housing does not move relative to the dispensing part on activation of the discharger, but rather the actuation device is configured to move the container from the storage state into the discharge state.

Advantageously the actuation device comprises an elastic member, in particular a spring, that is biased between the activation part and the actuation device or the container, preferably wherein the elastic member is connected to the activation part and is in particular integrally formed with the activation part. Using e.g. a spring as part of the activation device to bias the container relative to the housing provides a simple and reproducible form of activation device.

Optionally the elastic member is either formed at an outer wall of the activation part on an outer side of the activation part or is formed within the activation part at an inner wall of the activation part. In this way various kinds of activation devices can be made available.

It is preferred if the actuation device comprises at least one latching member that is configured to lock at least a part of the actuation device in the discharge state once the container has been moved into the discharge state, preferably wherein the latching member is configured to release the lock on opening the releasable connection present between the dispensing part and the activation part.

Through the provision of at least one such latching member one can ensure that the activation part and the dispensing part are fixed in position relative to one another once connected to one another and can thereby ensure the correct operation of the discharger.

The actuation device may comprise a ratchet mechanism, preferably wherein the ratchet mechanism is formed directly between the activation part and an outer wall of the container received and stored in the housing. A ratchet mechanism is a simple mechanism that can be used to reliably entrain one component, i.e. the container, relative to another component, i.e. the activation part. The ratchet mechanism may comprise the elastic member discussed in the foregoing.

It is preferred if the outer wall of the container comprises a linear rack with teeth, with the linear rack with teeth forming a part of the ratchet mechanism, preferably wherein:
- each tooth of the linear rack of teeth comprises first and second slopes, with the first slope being less steep than the second slope, and/or
- the teeth of the linear rack of teeth are uniform, but asymmetrical, and/or
- the linear rack of teeth extends circumferentially around the outer wall of the container. Such features further beneficially enhance the reliable operation of the ratchet mechanism as an actuation device.

Advantageously the activation part comprises at least one pawl, that in particular comprises the elastic member, and preferably comprises two pawls, with the at least one pawl forming a part of the ratchet mechanism, preferably wherein
- the activation part comprises at least one opening, preferably two openings, through which the at least one pawl engages the container; and/or
- the at least one pawl is configured to engage the linear rack with teeth. Forming the pawl of the activation part in this way further beneficially enhances the reliable operation of the ratchet mechanism. Moreover, by providing an opening in the activation part through which the pawl can engage the linear rack with teeth facilitates a direct interaction between the pawl and the container.

Preferably the activation part is configured and arranged so that it does not come into contact with a fluid stored in the container on discharging the fluid from the container in the discharge state.

The discharger may further comprise seals to seal between the dispensing part and the container arranged in the two-part housing, with the seal being arranged to avoid a fluid from coming into contact with the activation part. The seal may be arranged at the static piston, in particular at the piercing end of the static piston in order to avoid a leak of the fluid out of the container and into the compartment of the two-part housing.

Advantageously the container of the discharger is filled with a fluid selected from the group of members consisting of a topical medication, a medical fluid, a cosmetic and/or skin care preparation, a dental fluid, a veterinary fluid, an adhesive fluid, a disinfectant fluid, and combinations of the foregoing.

In this way, for example, bleaching agents for teeth, fluoride for teeth, disinfectants, adhesives, wound adhesives, topical anesthetics, sunscreen, after sun, skin moisturizers or other drugs and cosmetics can be stored in the discharger and administered using the discharger. Such fluids can e.g. be the aforementioned frontline, eye drops and nose drops used to e.g. decongest eyes and noses of patients suffering from allergies, colds or flues.

It is preferred if the container comprises first and second ends and a reservoir configured to store a fluid arranged within the container, the reservoir being sealed off by a seal, in particular a film, arranged within the container, the seal being set back from the first end of the container, with a fluid stored in the container being able to be discharged via said first end and said seal when the seal is pierced, optionally wherein at least a part of a piercing end of the static piston is arranged within the container at the first end between the first end and the seal in the storage state and the container is configured to be moved towards the static piston by means of the actuation device such that the piercing end of the static piston is moved to pierce the seal and further towards the second end of the container in the discharge state.

In this way an alignment of the two housing parts of the two-part housing with the container can be ensured permitting a simple assembly of the discharger with correct alignment of the parts. Moreover, the seal between the container and the static piston can in this way also be made available prior to activation of the discharger.

According to a further aspect the present invention relates to a container for a manually operated discharger in particular in accordance with the teaching presented herein, wherein the container comprises first and second ends and a reservoir configured to store a fluid arranged within the container, the reservoir being sealed off by a seal, in particular a film, arranged within the container, the seal being set back from the first end of the container, with a fluid stored in the reservoir of the container being able to be discharged via said first end and said seal when the seal is pierced, optionally wherein the container comprises an outer wall extending between the first end and the second end, with the outer wall comprising a linear rack with teeth, wherein the container is preferably filled with a fluid selected from the group of members consisting of a topical medication, a medical fluid, a cosmetic and/or skin care preparation, a dental fluid, a veterinary fluid, an adhesive fluid, a disinfectant fluid, and combinations of the foregoing.

Containers like prior art dischargers are single use components that following a dispensing of a fluid therefrom are simply discarded. Forming a container with a seal set back from the first end enables the provision of a single use container for use with a reusable activation part of a manually operated discharger.

According to a further aspect the present invention relates to a dispensing part for a manually operated discharger in as discussed herein, wherein the dispensing part comprises a part of a releasable connection configured to be connected to an activation part at a container end of the dispensing part, a static piston arranged at the container end and a discharge end arranged opposite to the container end, the discharge end comprising one of a dispensing connector and a dispenser, with the dispensing part in particular being configured for a single use.

Such dispensing parts, like containers are configured for single use and that following a dispensing of a fluid therefrom may simply discarded. The dispensing parts are beneficially configured to be used with a reusable activation part of a manually operated discharger.

According to a further aspect the present invention relates to an activation part for a manually operated discharger in particular as discussed herein, optionally in combination with the dispensing part as discussed herein, wherein the activation part comprises a part of a releasable connection at a dispensing end of the activation part configured to be connected to a dispensing part, the activation part comprising a reception space forming at least part of a compartment configured to removeably store a container within the manually operated discharger, such as the container discussed in the foregoing, the container being configured to store a fluid in the storage state of the discharger, the activating part further comprising an actuation device that is configured to cooperate with the container and that is configured to move the container from a storage state towards the dispensing end into a discharge state in which the fluid stored in the container can be discharged, with the activation part in particular being configured for multiple-uses.

The advantages discussed in the foregoing in connection with the activation part of the discharger also hold true with respect the present activation part.

According to a further aspect the present invention relates to a method of assembling and using a discharger, in particular in accordance with at least one of the preceding claims, the method comprising the steps of:
- providing a container, in particular as discussed herein;
- providing a dispensing part, in particular as discussed herein;
- providing an activation part having an actuation device at least a part of which being able to be moved relative to the activation part, in particular as discussed herein;
- inserting the container into the activation part and arranging it with respect to the actuation device;
- inserting a piercing end of the static piston of the dispensing part into a first end of the container without piercing a seal of the container;
   releasably connecting the dispensing part to the activation part, in particular by means of a releasable connection, optionally
- activating the actuation device to move the container from a storage state into a discharge state by moving the container towards the static piston and piercing the seal of the container; and further optionally
- discharging a fluid stored in the container from the discharger via the static piston.

The advantages associated with the discharger in accordance with the invention likewise hold true for the method described herein.

Further embodiments of the invention are described in the following description of the Figures. The invention will be explained in the following in detail by means of embodiments and with reference to the drawing in which is shown:
- Fig. 1a: a top view of a discharger in a storage state;
- Fig. 1b: a sectional view of the discharger of Fig. 1a along the sectional line A-A in a discharged state;
- Fig. 1c: an enlarged sectional view of view C of a first type of discharger shown in Fig. 1b;
- Fig. 1d: an enlarged sectional view of view E of a second type of discharger similar to the one shown in Fig. 1b;
- Fig. 1e: a side view of the discharger of Fig. 1a;
- Fig. 1f: a sectional view of the discharger of Fig. 1e along the sectional line B-B;
- Fig. 2a: a side view of a further type of discharger;
- Fig. 2b: a sectional view of the discharger of Fig. 2a along the sectional line A-A in the storage state;
- Fig. 2c: a top view of a further discharger similar to that of Fig. 2a;
- Fig. 2d: a sectional view of the discharger of Fig. 2c along the sectional line B-B in the storage state;
- Fig. 3a: a sectional view of a first type of container stored in the discharger of Fig. 2c along the sectional line C-C of Fig. 2d;
- Fig. 3b: a sectional view of a second type of container stored in the discharger of Fig. 2c along the sectional line C-C of Fig. 2d; and
- Fig. 3c: a sectional view of a third type of container stored in the discharger of Fig. 2c along the sectional line C-C of Fig. 2d.

In the following the same reference numerals will be used for parts having the same or equivalent function. Any statements made having regard to the direction of a component are made relative to the position shown in the drawing and can naturally vary in the actual position of application.

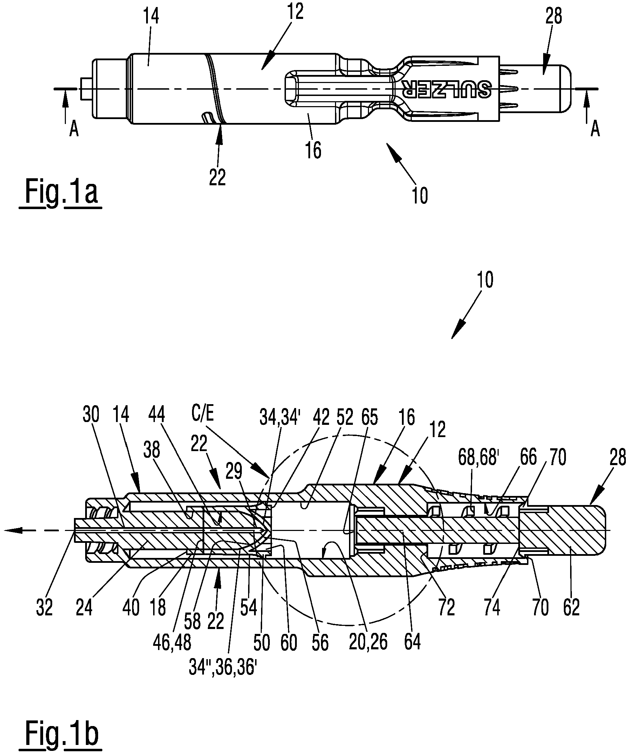

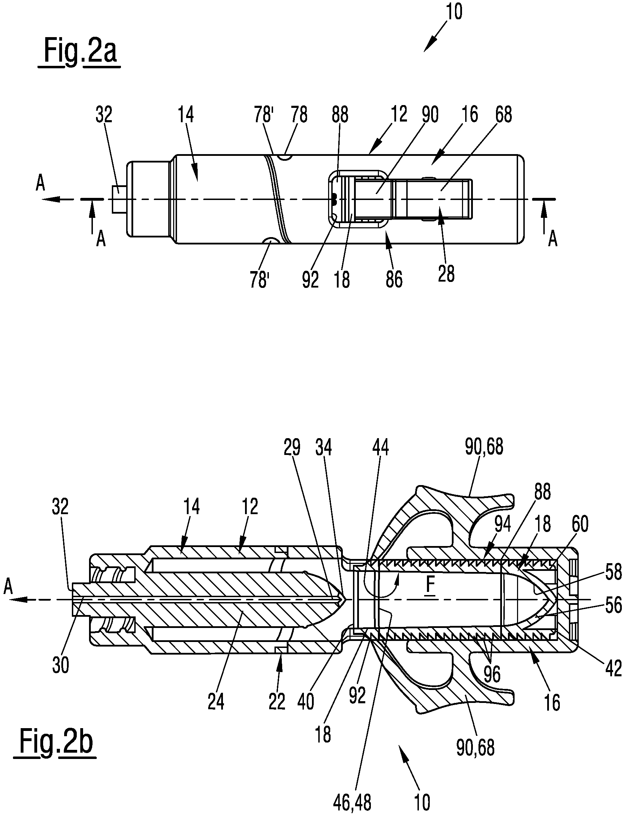

Fig. 1a shows a top view of a discharger 10 in a storage state. The discharger comprises a two-part housing 12 formed of a dispensing part 14 and an activation part 16. The two part housing 12 is configured to receive a container 18 (see Fig. 1b). The container 18 is configured to store a fluid F (see Fig. 2b) in a storage state of the discharger 10.

The container 18 is stored within a compartment 20 formed between the dispensing and activation parts 14, 16. The dispensing part 14 and the activation part 16 are configured to be releasably coupled to one another by means of a releasable connection 22.

As indicated in Fig. 1b the dispensing part 14 comprises a static piston 24 and the activation part comprises a reception space 26 forming at least part of the compartment 20 that is configured to removably store the container 18 within the discharger 10.

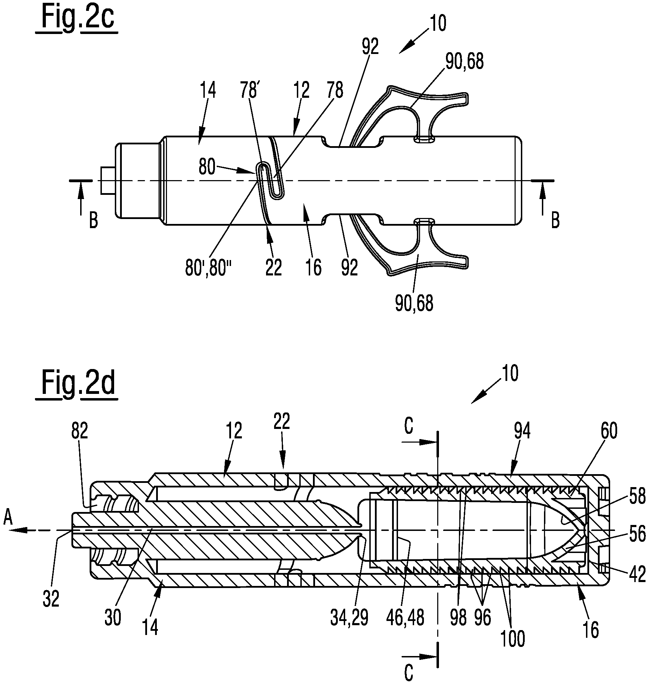

The discharger 10 further comprises an actuation device 28 that cooperates with the container 18 and that is configured to move the container 18 from the storage state in which the container 18 is received in the compartment 20 (see e.g. Figs. 2b and 2d) into a discharge state in which container is moved out of the compartment 20 and over the static piston 24 (see e.g. Fig. 1b). On moving the container 18 over the static piston 24, the fluid stored F in the container 18 can be discharged via the static piston 24.

As also indicated in the sectional drawing of Fig. 1b the static piston 24 is arranged within the housing 12 of the discharger 10.

The static piston has an inlet 29 that is arranged within the housing 12. The inlet 29 is connected to an outlet 32 of the discharger 10 in a fluid conducting manner. For this purpose the inlet 29 leads into a passage 30 that connects the inlet 29 with the outlet 32. The passage 30 thereby extends through the static piston 24 and the discharger 10. The fluid stored F in the container 18 can hence be discharged via the passage 30 of the static piston 24

The discharger 10 is preferably designed such that the only way a fluid present in the container 18 can pass to the outside is via the inlet 29, the passage 30 and the outlet 32.

The static piston 24 comprises a piercing tip 34 having the inlet 29 formed at one end 34' of the piercing tip 34. The other end 34" of the piercing tip 34 comprises a sealing lip 36 as a sealing element 36', i.e. the sealing element 36' is arranged at the static piston 24.

Further sealing elements (not shown) can likewise be formed at the static piston 24, for example, second and third sealing elements could additionally be provided. The first, second and third sealing lips 36 could then be arranged one after the other in parallel to one another in a direction of a longitudinal axis A of the static piston 24 that extends along the passage 30.

The sealing element 36' is configured to cooperate with an inner wall 38 of the container 18. The sealing element 36' thereby provides a seal between the dispensing part 14 and the container 18 arranged in the two-part housing 12. The sealing element 36' is arranged within the housing 12 to avoid a fluid F from coming into contact with the activation part 16.

In this way the discharger 10 is configured and arranged so that the activation part 16 thereof does not come into contact with a fluid F stored in the container 18 on discharging the fluid F from the container 18 in the discharge state.

In this connection it should be noted that the container 18 can be filled with a fluid F selected from the group of members consisting of a topical medication, a medical fluid, a cosmetic and/or skin care preparation, a dental fluid, a veterinary fluid, an adhesive fluid, a disinfectant fluid, and combinations of the foregoing.

As further illustrated in the sectional view of Fig. 1b taken of the discharger of Fig. 1a along the sectional line A-A, the container 18 comprises first and second ends 40, 42 and a reservoir 44 configured to store the fluid F. The reservoir 44 is arranged within the container 18 and is sealed off by a seal 46. In the present example the seal is a film 48.

The seal 46 is arranged within the container 18 and is set back from the first end 40 of the container 18. The fluid F stored in the reservoir 44 of the container 18 is able to be discharged via said first end 40 when the seal 46 is pierced.

The container 18 has a projection 50 formed at an outer surface 54 thereof. The projection 50 in the example shown is present at the second end 42. The projection 50 could be arranged at any height along the outer surface 54 of the container 18.

The projection 50 is namely provided to circumferentially extend around the preferably cylindrical shape of the container 18 in order to provide a guide at the container 18 by means of which this can be guided relative to an internal wall 52 of the activation part 16 from the storage state into the discharge state.

It is optionally possible that at least a part of the piercing end 34' of the static piston 24 is arranged within the container 18 at the first end 40 between the first end 40 and the seal 46 in the storage state. In any event the container 18 is configured to be moved towards the static piston 24 by means of the actuation device 28 such that the seal 46 is moved towards the piercing end 34' of the static piston 24 to pierce the seal 46 and further towards the second end 42 of the container 18 in the discharge state.

The seal 46 is set back from the first end 40 of the container 18 by approximately 30% of a length of the container 18. In this connection it should be noted that the seal 46 can be set back from the first end 40 by at least 5%, in particular by 5 to 40%, of the length of the container in the direction of the longitudinal axis.

By arranging the piercing end 34' in particular together with the sealing element 36' within the container 18 at the front end 40 enables the seal between the static piston 24 and the container 18 to be aligned prior to use of the discharger 10 and hence leads to a further avoidance of the risk of unwanted leaks.

As further indicated in Fig. 1b, the reservoir 44 has an internal shape at the second end 42 that is complementary to an outer shape of the piercing tip 34. In this way one can ensure that as much as possible of the fluid F stored in the container 18 can be discharged from the container 18. For this purpose the reservoir 44 has a piercing tip receiving end 56 at the second end 42.

The piercing tip receiving end 56 is formed as an extension of the inner wall 38 by a wall 58 that converges from the inner wall 38 to a common point at the piercing tip receiving end 56 that coincides with the longitudinal axis A. This means that an internal diameter of the container 18 reduces from the inner wall 38 to the longitudinal axis A along the wall 58.

As also shown the piercing tip receiving end 56 is set back from the second end 42 of the container 18 by approximately 10% of a length of the container 18. In this connection it should be noted that the piercing tip receiving end 56 can be set back from the second end 42 by at least 5%, in particular by 5 to 20%, of the length of the container in the direction of the longitudinal axis.

A web 60 is provided in the region of the second end 42 of the container 18. The web 60 extends in parallel to the wall 38 between the first and second ends 40, 42 of the container 18. This web 60 may form part of a base at the second end 42. The projection 50 may beneficially be formed to project from the web 60.

It should be noted in this connection that the web 60 may also be arranged inclined with respect to the wall 38, this means that the web 60 could taper away from the internal wall 52 of the reception space 26. Alternatively the web 60 could be arranged to taper towards the internal wall 52 of the reception space 26.

A size of the reservoir 44 defines the volume of fluid F that can be stored in the container 18. This means that if a lesser volume of fluid F is to be stored within the reservoir 44, then e.g. a length of the reservoir 44 in the direction of the longitudinal axis A can be selected shorter. Consequently, if a greater volume of fluid F is to be stored in the container 18 then a length of the reservoir 44 can be selected longer than in the present case.

In this connection it should be noted that typical filling volumes of the reservoir 44 of the container 18 are 0.1 to 10 ml, preferably 0.2 to 5 ml.

It should further be noted that a thickness of the wall 38 of the reservoir 44 is typically selected in the range of 0.7 to 1.5 mm, preferably in the range of 0.9 to 1.1 mm and especially of around 1 mm. Likewise a thickness of the outer wall 14 of the container 4 is typically selected in the range of 0.7 to 1.5 mm, preferably in the range of 0.9 to 1.1 mm and especially of around 1 mm.

It should be noted in this connection that if a reservoir 44 of greater volume is selected then a length of the static piston 34 can also be increased in order to ensure that as much as possible of the fluid F initially stored in the reservoir 44 is discharged from the discharger 10.

The actuation device 28 shown comprises a button 62 that is configured to be pressed in the direction of the longitudinal axis A to bring about a movement of the container 18 from the storage state into the discharge state. The movement of the container 18 is brought about by a plunger 64 that extends from a bottom end 74 of the button in the direction of the longitudinal axis A.

On entraining the container 18, an end 65 of the plunger 64 is in contact with the container 18 and guides this into the discharge state. Thus, at least a part of the actuation device 28, namely the button 62 and the plunger 64 are moved relative to the activation part 16.

The actuation device 28 further comprises an elastic member 68 that is provided in a receiving space 66. The elastic member is a spring 68' that is biased between the activation part 16 and the button 62. More specifically the spring is biased between a ledge 72 of the receiving space 66 and the end 74 of the button 62.

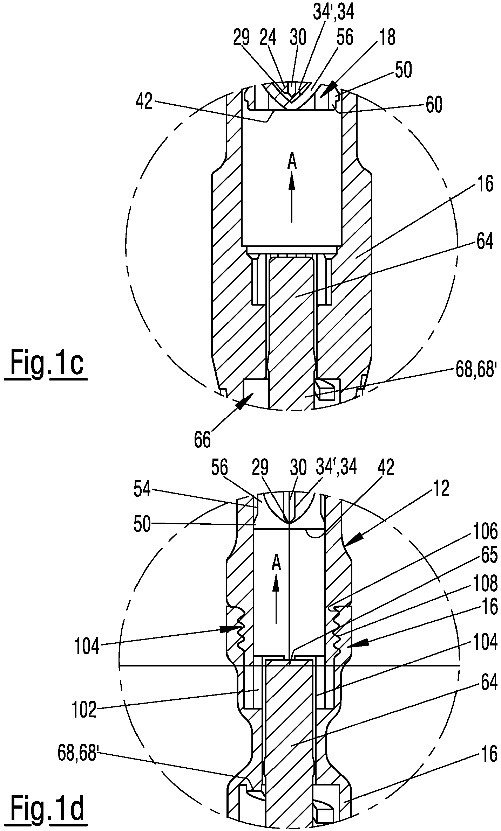

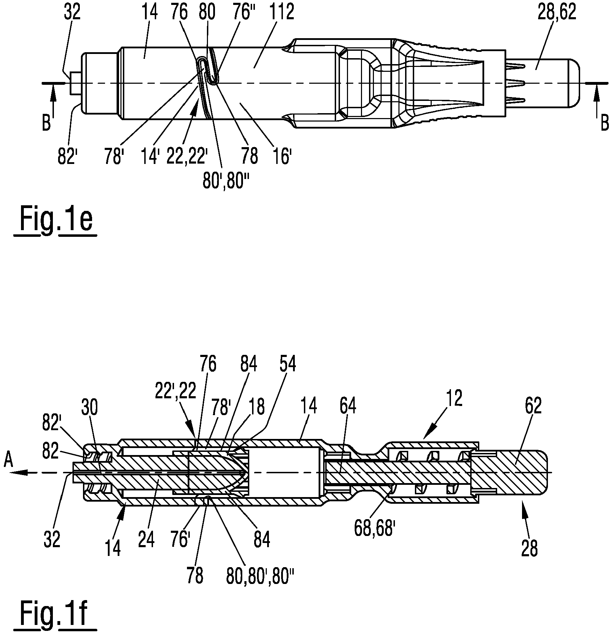

The spring 68' can be connected to the activation part 16 and can, for example, be integrally formed with the ledge 72 of the activation part 16 (see e.g. Figs. 1d and 1f). Alternatively the spring 68' can be a separate component that is inserted into the receiving space 66 on assembly of the activation part 16 (see e.g. Fig. 1c).

The button 62 has a hook member 70 at the end 74. The hook member 70 is provided to engage a wall of the activation part 16 present in the receiving space 66. The hook member 70 acts as a snap-in connection to lock the button 62 with the plunger 64 to the activation part 16 in order to form the actuation device 28.

The button 62 and plunger 64 cooperate with the actuation part 16 in a manner like the button and spring arrangement of a spring loaded ball point pen.

Fig. 1c shows an enlarged sectional view of view C of a first type of discharger 10 as shown in Fig. 1b. After releasing the button 62 and having moved the button and the plunger 64 in the longitudinal axis A, the spring 68' causes the button 62 and the plunger 64 to move back in the opposite direction until the hook member 70 engages the end of the activation part 16.

Fig. 1d shows an enlarged sectional view of view E of a second type of discharger 10 similar to the one shown in Fig. 1b. The difference to the view of Fig. 1d is that the releasable connection 22 is formed from a threaded connection 104.

The threaded connection 104 comprises an internal thread 106 at the activation part 16 and an external thread 108 at the dispensing part 14. Vice versa, the internal thread 106 could similarly be provided at the dispensing part 14 and the external thread 108 could be provided at the activation part 16.

Moreover, like the activation mechanism in a spring loaded ball point pen, the discharger 10 of Fig. 1d is configured such that the plunger 64 may remain in the discharge state after discharging the fluid F previously stored in the container 18, i.e. in a position in which the plunger 64 is moved along the longitudinal axis A and out of the storage state.

For this purpose the actuation device comprises a latching member 102 that is configured to lock at least a part of the plunger 64 in the discharge state once the container 18 has been moved into the discharge state.

On moving the plunger 64 into the discharge state, the plunger 64 engages the latching member 102 and thereby pushes the latching member 102 radially outwardly such that the latching member 102 is biased between the housing 12, in the embodiment shown against the dispensing part 14, and the plunger 64. Due to the radially acting bias the plunger 64 is not able to be moved in the axial direction back towards the storage state.

The latching member 102 is only arranged at the end 65 of the plunger 64 in order to avoid the plunger 64 from becoming stuck in position at a different part within the housing 12.

The latching member 102 can either be formed from a single component or from a multiple number of components. For example 3 or 4 wedge shaped components of like design can be arranged at the end 65 to form the latching member 102.

On opening the discharger 10, i.e. on opening the releasable connection 22 present between the dispensing part 14 and the activation part 16, such that one can discard the container 18 and the dispensing part 14 after use, the bias exerted by the latching member 102 between the housing 12 and the plunger 64 is released to release the lock on the activation device 28.

Fig. 1e shows a side view of the discharger of Fig. 1a. In the region of the outlet 32 the dispensing part 14 comprises a Luer lock type of connection 82 (see Fig. 1f) as a form of dispenser connector by means of which different kinds of dispensers can be connected to the dispensing part 14. The different types of dispensers then respectively form the outlet of the discharger 10.

Alternatively hereto the dispenser can be integrally formed at the dispensing part 14 such that the outlet 32 from the dispensing part 14 also forms the outlet of the discharger 10 at a discharge end 82' of the discharger 10.

In this connection it should be noted that various types of dispensers could be connected to or integrally formed with the dispensing part 14. By way of example the following types of dispensers could be arranged at the outlet 32 of dispensing part 14, namely leaf-like applicators, spatula-like applicators, flocked type applicators, micro-brush applicators, micro-bristle applicators, needle type dispensers, sponge-like dispensers, comb-like dispensers, cannula type dispensers etc.

In the examples shown herein the releasable connection 22 is formed by a plug and rotate type of connection 22'. Alternatively the releasable connection could be formed by a bayonet type of connection or the threaded connection 104. The releasable connection is formed between a container end 14' of the dispensing part 14 and the discharge end 16' of the activation part 16.

A first part of the releasable connection 22 is shown in Fig. 1e and comprises two slots 76, 76' respectively present at the dispensing part 14 and the activation part 16. A similar arrangement is provided at the other side of the discharger 10.

The respective parts of the slots 76, 76' of the dispensing and activation parts 14, 16 cooperate with one another and form the releasable connection 22 between the dispensing and activation parts 14, 16. An arm 78 (or wall) of the dispensing part 14 engages the slot 76' of the activation part 16. Similarly an arm 78' (or wall) of the activation part engages the slot 76 of the activation part.

The slots 76, 76' and hence the arms 78, 78' are inclined with respect to the longitudinal axis A of the discharger 10. On assembling the discharger 10 the slots 76, 76' and arms 78, 78' are respectively aligned with one another and the activation part 16 and the dispensing part 14 are moved axially towards one another, thereafter the activation part 16 and the dispensing part 14 are rotated with respect to one another to form the releasable connection 22.

In order to secure the activation part 16 at the dispensing part 14 a latched connection 80 is present between the dispensing and activation parts 14, 16 to fix the dispensing and activation parts 14, 16 one to another. The latched connection 80 is formed as part of the releasable connection 22.

The latched connection 80 is formed by a recess 80' formed in the slot 76 of the dispensing part that cooperates with a projection 80" formed at the arm 78' of the activation part. The projection 80" could also be formed at the arm 78 of the dispensing part 14 and the recess correspondingly at the slot 76' of the activation part 16. Alternatively both slots 76, 76' could comprise a respective recess 80' or projection 80" or alternatingly a recess 80' and a projection 80" to form the latched connection 80.

Fig. 1f shows a sectional view of the discharger of Fig. 1e along the sectional line B-B. A Luer lock type of connection 82 can be seen that surrounds the outlet 32 and the passage 30 in the region of the outlet 32.

Fig. 1f also shows that the container 18 has a connection line 84 via which the first and second ends 40, 42 of the container 18 are connected one to another. Since the container has the seal 46 that is set back from the first end 40, the seal 46 cannot be attached to the container 18 once this has been filled as is the case with prior art containers, but rather the container is composed of two parts that are connected one to another at the connection line 84 once the container 18 has been filled with the fluid F.

The container 18 may also comprise a different manner in which it can be filled with the fluid F. By way of example an inlet (not shown) that is sealed off after filling may be provided at the second end 42. Other ways of filling the container 18 can easily be envisaged by the person skilled in the art.

Fig. 2a shows a side view of a further type of discharger 10. The actuation device 28 comprises a ratchet mechanism 86. Part of the ratchet mechanism 86 is formed directly between the activation part 16 and an outer wall 88 of the container 18 received and stored in the housing 12.

The activation part 16 comprises two elastic members 68 that are respectively formed as part of a respective pawl 90 of the ratchet mechanism 86. The activation part 16 further comprises two openings 92 at either side of the discharger 10. The two pawls respectively pass through the two openings 92 and engage the container 18 that is accessible through the openings 92.

Fig. 2b shows a sectional view of the discharger 10 of Fig. 2a along the sectional line A-A in the storage state, i.e. in the state in which the container 18 has not been moved towards the static piston 24 and in which the seal 46 has not yet been pierced. The two pawls 90 are respectively configured to engage a linear rack 94 with teeth 96 forming a further part of the ratchet mechanism 86.

Fig. 2c shows a top view of the further discharger of Fig. 2a. The actuation device 28 shown in Fig. 2 thus comprises a lever in the form of the pawl 90 that is configured to be pressed to bring about a movement of the container 18 from the storage state into the discharge state. Also in this example of the discharger 10 is a part of the actuation device 28 moved relative to the activation part 16 in order to bring about a movement of the container along the longitudinal axis A towards the piercing tip 34.

Fig. 2d shows a sectional view of the discharger of Fig. 2c along the sectional line B-B in the storage state. The outer wall 88 of the container 18 comprises the linear rack 94 with teeth 96. Each tooth 96 of the linear rack 94 of teeth 96 comprises first and second slopes, with the first slope being less steep than the second slope. In this way the teeth 96 of the linear rack 94 of teeth 96 are uniform, but asymmetrically arranged.

As indicated also in Fig. 2d the linear rack 94 of teeth 96 extends circumferentially around the outer wall 88 of the container 18 between the first and second ends 40, 42.

A ratchet is a mechanical device that allows continuous linear motion in only one direction while preventing motion in the opposite direction. The ratchet shown herein consists of the linear rack 94 with teeth 96, and a pivoting, spring-loaded finger, namely the pawl 90. The teeth 96 are uniform but asymmetrical, with each tooth 96 having a moderate slope on one edge and a much steeper slope on the other edge.

When the teeth 96 are moving in the unrestricted direction, i.e. along the longitudinal axis A towards the piercing tip 34, the pawl 90 easily slides up and over the edges of the teeth 96 having the moderate slope, with the elastic member 68 forcing the pawl into a depression 98 between the teeth 96 as it passes the tip 100 of each tooth 96. When the teeth 96 move in the opposite direction, i.e. away from the piercing tip 34, however, the pawl 90 will catch against the steeply sloped edge of the first tooth 96 it encounters, thereby locking it against the tooth 96 and preventing any further motion in that direction.

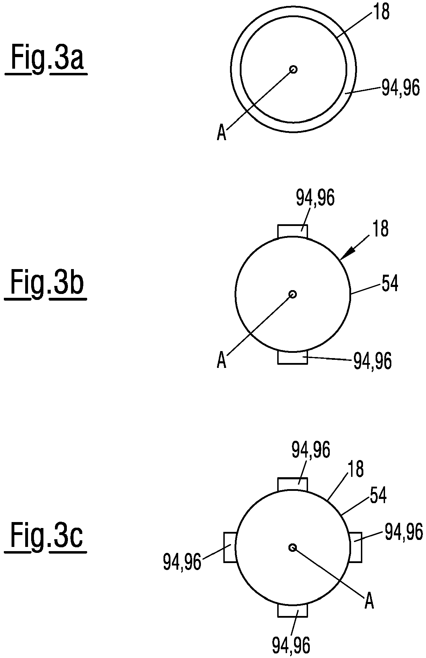

Figs. 3a to 3c show respective sectional views of first, second and third types of containers 18 that may be stored in the discharger 10 shown in Fig. 2d. The sectional view is taken along the sectional line C-C of Fig. 2d. The first type of container shown in Fig. 3a has the linear rack 94 with teeth 96 extending circumferentially around the container 18 as discussed in the foregoing.

Fig. 3b shows a second type of container 18 having two linear racks 94 with teeth 96. The linear racks 94 with teeth 96 are arranged opposite one another at the outer surface 54 of the container 18.

Fig. 3c shows a third type of container 18 having four linear racks 94 with teeth 96. The four linear racks 94 are arranged in a cross-like manner at 90 degrees with respect to one another and the longitudinal axis A at the outer surface 54 of the container 18.

Thus, the discharger 10 discussed in the foregoing is made up of three essential components, namely the container 18 that is configured to store the fluid F, the dispensing part 14 via which the fluid can be discharged from the container 18 and the activation part 16 that brings about a movement of the container relative to the static piston 24 in order to enable the piercing of the film 48 and a subsequent dispensing of the fluid from the container 18.

In use of the components these can be assembled to provide a discharger in the storage state. Following the assembly the discharger 10 can be used.

On assembling the discharger the following stops are carried out:
- providing the container 18 with a fluid F stored therein;
- providing the dispensing part 14;
- providing the activation part 16;
- inserting the container 18 into the activation part 16 and arranging it with respect to the actuation device 28;
- inserting the piercing end 34' of the static piston 24 of the dispensing part 14 into the first end 40 of the container 18 without piercing the seal 46 of the container 18;
- releasably connecting the dispensing part 14 to the activation part 16 by aligning the slots 76 and arms 78 of the dispensing part 14 relative to the slots 76' and arms 78' of the activation part 16 and axially and rotationally moving the dispensing part 14 to the activation part 16 relative to one another.

On activating the discharger 10 the actuation device is 28 actuated and the actuation device 28 moves the container 18 from the storage state into a discharge state by moving the container 18 towards the static piston 24 and piercing the seal 46 of the container 18. Following the piercing of the seal 46, the fluid F stored in the container 18 is discharged via the passage 30 of the static piston 24 towards and out of the outlet 32. The fluid F can then be applied at the respective site of application.

### List of reference numerals:

- 10: discharger
- 12: housing
- 14, 14': dispensing part, container end
- 16, 16': activation part, dispensing end
- 18: container
- 20: compartment
- 22, 22': releasable connection, plug and rotate type of connection
- 24: static piston
- 26: reception space
- 28: actuation device
- 29: inlet
- 30: passage
- 32: outlet
- 34, 34', 34": piercing tip, piercing end, other end of 34
- 36, 36': sealing lip, sealing element
- 38: inner wall
- 40: first end of 18
- 42: second end of 18
- 44: reservoir
- 46: seal
- 48: film
- 50: projection at 18
- 52: internal wall of 16
- 54: outer surface
- 56: piercing tip receiving end
- 58: wall
- 60: web
- 62: button
- 64: plunger
- 66: receiving space
- 68, 68': elastic member, spring
- 70: hook member
- 72: ledge
- 74: end of 62
- 76, 76': slot at 14, slot at 16
- 78, 78': arm (wall) at 14, arm (wall) at 16
- 80, 80', 80": latched connection, recess, projection
- 82, 82': Luer lock type of connection, discharge end
- 84: connection line
- 86: ratchet mechanism
- 88: outer wall of 18
- 90: pawl
- 92: opening
- 94: linear rack
- 96: tooth
- 98: depression
- 100: tip
- 102: latching member
- 104: threaded connection
- 106: internal thread
- 108: external thread

- A: longitudinal axis
- F: fluid

## Claims

1. A discharger (10) comprising a two-part housing (12) formed of a dispensing part (14) and an activation part (16) and further comprising a container (18) configured to store a fluid (F) in a storage state of the discharger (10), the container (18) being stored within a compartment (20) formed between the dispensing and activation parts (14, 16), wherein the dispensing part (14) and the activation part (16) are configured to be releasably coupled to one another by means of a releasable connection (22), with the dispensing part (14) comprising a static piston (24) and the activation part (16) comprising a reception space (26) forming at least part of the compartment (20) configured to removably store the container (18) in the storage state of the discharger (10), the discharger (10) further comprising an actuation device (28) that cooperates with the container (18) and that is configured to move the container (18) from the storage state into a discharge state in which the fluid (F) stored in the container (18) can be discharged via the static piston (24).

2. A discharger (10) in accordance with claim 1, wherein the releasable connection (22) is formed by at least one of a bayonet type of connection, a plug and rotate type of connection (22'), a Luer Lock type of connection, and a threaded connection (104).

3. A discharger (10) in accordance with claim 1 or claim 2, wherein the dispensing part (14) and the activation part (16) each comprise two slots (76, 76'), with respective parts of the slots (76, 76') of the dispensing and activation parts (14, 16) cooperating with one another and forming the releasable connection (22) between the dispensing and activation parts (14, 16), optionally wherein the slots (76, 76') are inclined with respect to a longitudinal axis (A) of the discharger (10) that is aligned with the static piston (24); and/or
wherein a latched connection (80) is present between the dispensing and activation parts (14, 16) to fix the dispensing and activation parts (14, 16) one to another, wherein the latched connection (80) is in particular present at the releasable connection (22).

4. A discharger (10) in accordance with claim 3, wherein at least one of the slots (76, 76') of the dispensing and activation parts (14, 16) comprises a recess (80') in one of its walls (78, 78') cooperating with a projection (80") formed at a wall (78, 78') of the respective other one of the dispensing and activation parts (14, 16) to form the latched connection (80).

5. A discharger (10) in accordance with at least one of the preceding claims, wherein the actuation device (28) comprises at least one button (62) or lever (90) that is configured to be pressed to bring about a movement of the container (18) from the storage state into the discharge state, preferably wherein the container (18) remains in the discharge state after discharging the fluid (F) previously stored in the container (18); and/or
wherein at least a part of the actuation device (28) can be moved relative to the activation part (16).

6. A discharger (10) in accordance with at least one of the preceding claims, wherein the actuation device (28) comprises an elastic member (68), in particular a spring (68'), that is biased between the activation part (16) and the actuation device (28) or the container (18), preferably wherein the elastic member (68) is connected to the activation part (16) and is in particular integrally formed with the activation part (16); and optionally wherein the elastic member (68) is either formed at an outer side of the activation part (16) or is formed within the activation part (16).

7. A discharger (10) in accordance with at least one of the preceding claims, wherein the actuation device (28) comprises a latching member (102) that is configured to lock at least a part of the actuation device (28) in the discharge state once the container (18) has been moved into the discharge state, preferably wherein the latching member (102) is configured to release the lock on opening the releasable connection (22) present between the dispensing part (14) and the activation part (16).

8. A discharger (10) in accordance with at least one of the preceding claims, wherein the actuation device (28) comprises a ratchet mechanism (86), preferably wherein the ratchet mechanism (86) is formed directly between the activation part (16) and an outer wall of the container (18) that is received and stored in the housing (12).

9. A discharger (10) in accordance with claim 8, wherein the outer wall (88) of the container (18) comprises a linear rack (94) with teeth (96), with the linear rack (94) with teeth (96) forming a part of the ratchet mechanism (86), preferably wherein:
- each tooth (96) of the linear rack (94) of teeth (96) comprises first and second slopes, with the first slope being less steep than the second slope, and/or
- the teeth (96) of the linear rack (94) of teeth (96) are uniform, but asymmetrical, and/or
- the linear rack (94) of teeth (96) extends circumferentially around the outer wall (88) of the container (18).

10. A discharger (10) in accordance with claim 8 and/or claim 9, in particular when dependent on claim 6, wherein the activation part (16) comprises at least one pawl (90), that in particular comprises the elastic member (68), and preferably comprises two pawls (90), with the at least one pawl (90) forming a part of the ratchet mechanism (86), preferably wherein
- the activation part (16) comprises at least one opening (92), preferably two openings (92), through which the at least one pawl (90) is configured to engage the container (18); and/or
- the at least one pawl (90) is configured to engage the linear rack (94) with teeth (96).

11. A discharger (10) in accordance with at least one of the preceding claims, wherein the activation part (16) is configured and arranged so that it does not come into contact with the fluid (F) stored in the container (18) on discharging the fluid (F) from the container (18) in the discharge state; and/or wherein seals (36) are provided to seal between the dispensing part (14) and the container (18) arranged in the two-part housing (12), with the seal (36) being arranged to avoid a fluid from coming into contact with the activation part (16), preferably wherein the seal (96) is arranged at the static piston (24); and/or
wherein the container (18) is filled with a fluid (F) selected from the group of members consisting of a topical medication, a medical fluid, a cosmetic and/or skin care preparation, a dental fluid, a veterinary fluid, an adhesive fluid, a disinfectant fluid, and combinations of the foregoing.

12. A discharger (10) in accordance with at least one of the preceding claims, wherein the container (18) comprises first and second ends (40, 42) and a reservoir (44) configured to store the fluid (F) arranged within the container (18), the reservoir being sealed off by a seal (46), in particular a film (48), arranged within the container (18), the seal (46) being set back from the first end (40) of the container (18), with the fluid (F) stored in the container (18) being able to be discharged via said first end (40) and said seal (46) when the seal (46) is pierced, optionally wherein at least a part of a piercing end (34') of the static piston (24) is arranged within the container (18) at the first end (40) between the first end (40) and the seal (46) in the storage state and the container (18) is configured to be moved towards the static piston (24) by means of the actuation device (28) such that the piercing end (34') of the static piston (24) is moved to pierce the seal (46) and further towards the second end (42) of the container (18) in the discharge state.

13. A container (18) for a manually operated discharger (10) in particular in accordance with at least one of the preceding claims, wherein the container (18) comprises first and second ends (40, 42) and a reservoir (44) configured to store a fluid (F) arranged within the container (18), the reservoir (44) being sealed off by a seal (46), in particular a film (48), arranged within the container (18), the seal (46) being set back from the first end (40) of the container (18), with the fluid (F) stored in the reservoir (44) being able to be discharged via said first end (40) and said seal (46) when the seal (46) is pierced, optionally wherein the container (18) comprises an outer wall (88) extending between the first end and the second ends (40, 42), with the outer wall (88) comprising a linear rack (94) with teeth (96), wherein the container (18) is preferably filled with a fluid (F) selected from the group of members consisting of a topical medication, a medical fluid, a cosmetic and/or skin care preparation, a dental fluid, a veterinary fluid, an adhesive fluid, a disinfectant fluid, and combinations of the foregoing.

14. A dispensing part (14) for a manually operated discharger (10) in particular in accordance with at least one of the preceding claims 1 to 12, wherein the dispensing part (14) comprises a part of a releasable connection (22) configured to be connected to an activation part (16) at a container end (14') of the dispensing part (14), a static piston (24) arranged at the container end and a discharge end (82') arranged opposite to the container end (14'), the discharge end (82') comprising one of a dispensing connector (82) and a dispenser, with the dispensing part (14) in particular being configured for a single use.

15. An activation part (16) for a manually operated discharger (10) in particular in accordance with at least one of the preceding claims 1 to 12, optionally in combination with the dispensing part (14) of claim 14, wherein the activation part (16) comprises a part of a releasable connection (22) at a dispensing end (16') of the activation part (16) configured to be connected to a dispensing part (14), the activation part (16) comprising a reception space (26) forming at least part of a compartment (20) configured to removeably store a container (18) within the manually operated discharger (10), such as the container (18) of claim 13, the container (18) being configured to store a fluid (F) in the storage state of the discharger (10), the activation part (16) further comprising an actuation device (28) that is configured to cooperate with the container (18) and that is configured to move the container (18) from a storage state towards the dispensing end (16') into a discharge state in which the fluid (F) stored in the container (18) can be discharged, with the activation part (16) in particular being configured for multiple-uses.

16. A method of assembling and using a discharger (10), in particular in accordance with at least one of the preceding claims 1 to 12, the method comprising the steps of:
- providing a container (18), in particular in accordance with claim 13;
- providing a dispensing part (14), in particular in accordance with claim 14;
- providing an activation part (16) having an actuation device (28) at least a part of which being able to be moved relative to the activation part (16), in particular in accordance with claim 15;
- inserting the container (18) into the activation part (16) and arranging it with respect to the actuation device (28);
- inserting a piercing end (34') of the static piston (24) of the dispensing part (14) into a first end of the container (18) without piercing a seal (46) of the container (18);
releasably connecting the dispensing part (14) to the activation part (16), in particular by means of a releasable connection (22), optionally
- activating the actuation device (28) to move the container (18) from a storage state into a discharge state by moving the container (18) towards the static piston (24) and piercing the seal (46) of the container (18); and further optionally
- discharging a fluid (F) stored in the container (18) from the discharger (10) via the static piston (24).
